# EUROPEAN PATENT SPECIFICATION

(11) **EP 2 366 407 B1**
(45) Date of publication and mention of the grant of the patent: **12.11.2014**
(21) Application number: 11165830.8
(22) Date of filing: 03.09.2004
(51) Int. Cl.: A61K 45/06, A61P 37/08

(54) **External preparation for percutaneous administration containing nonsteroidal anti-inflammatory analgesic**
Topisches Präparat für die perkutane Verabreichung enthaltend nichtsteroidale antiphlogistische Analgetika
Préparation à usage externe destinée à une administration percutanée et contenant un analgesique anti-inflammatoire non stéroïdique

(30) Priority: 03.09.2003 JP 2003311904
(43) Date of publication of application: 21.09.2011
(62) Divisional of application: 04772768.0
(73) Proprietor: HISAMITSU PHARMACEUTICAL CO., INC., Saga 841-0017 (JP)
(72) Inventor: Yoshitake, Kazuhisa, Tsukuba-shi Ibaraki 3050856 (JP); Atarashi, Kenji, Tsukuba-shi Ibaraki 3050856 (JP); Kuwahara, Tetsuji, Tsukuba-shi Ibaraki 3050856 (JP); Ikesue, Koichi, Tsukuba-Shi Ibaraki 3050856 (JP); Sakai, Michinori, Tsukuba-Shi Ibaraki 3050856 (JP); Hashimoto, Yoshiaki, Tosu-shi Saga 8410017 (JP); Tsuruda, Kiyomi, Tosu-shi Saga 8410017 (JP)
(74) Representative: Fyfe, Fiona Allison Watson

(56) References cited:
- EP-A1- 1 234 573
- EP-A1- 1 269 999
- EP-A1- 1 698 330
- US-A- 5 000 945
- US-A1- 2003 124 070
- RADSCHUWEIT ET AL: "UV-induces formation of hydrogen peroxide based on the photochemistry of ketoprofen", PHOTOCHEMISTRY AND PHOTOBIOLOGY, WILEY-BLACKWELL PUBLISHING, INC, US, vol. 73, no. 2, 1 February 2001 (2001-02-01), pages 119-127, XP002985755, ISSN: 0031-8655, DOI: DOI:10.1562/0031-8655(2001)073&LT,0119:UIF OHP&GT,2.0.CO,2

## Description

### Technical Field

The present invention relates to an external preparation for percutaneous administration containing ketoprofen as photosensitive nonsteroidal anti-inflammatory drug and 4-*tert*-butyl-4'-methoxydibenzoylmethane as UVA blocker, wherein the proportion of 4-*tert*-butyl-4'-methoxydibenzoylmethane as UVA blocker is 0.5 to 20 wt % relative to the entire amount of the composition..

### Background Art

Since ketoprofen has excellent anti-inflammatory action and analgesic action, they are contained as medicinal components in various forms of external preparations for percutaneous administration, such as patches, for example poultices and plasters, gels, creams, ointments, and liniments. However, some of the external preparations for percutaneous administration of ketoprofen are photosensitive, and very occasionally the development of photosensitive dermatitis has been reported as a side effect.

As attempts to suppress the photosensitive dermatitis, there have been reported a case in which formation of a photolyte is suppressed by adding a UV absorber to a ketoprofen-containing external preparation, thus preventing photolysis of ketoprofen (ref. Patent Publication 1), a case in which a support of a patch containing an NSAID is subjected to UV shielding (ref. Patent Publication 2), and a case in which titanium oxide is added to an anti-inflammatory external skin preparation (ref. Patent Publication 3), but it cannot be said that any thereof sufficiently prevents the development of photosensitive dermatitis, and there is a desire for still further improvement in these preparations.
[Patent Publication 1] JP, A, 60-155111
[Patent Publication 2] WO 01/68061
[Patent Publication 3] JP, A, 9-169658
[Patent Publication 4] JP, A, 53-99316
[Patent Publication 5] JP, A, 56-22711
[Patent Publication 6] JP, A, 2000-136122

Additional prior art includes EP 1234573 where is described a topical pharmaceutical composition for application to the skin or mucosa containing at least one non-steroidal anti-inflammatory as active ingredient; at least one ultraviolet radiation filter; and at least one inert excipient or carrier.

US 2003/0124070 describes a photostable and synergistically enhanced topical sunscreen composition. There is also provided a method of enhancing the photostability of a sunscreen active in a topical sunscreen composition and a method of synergistically enhancing the UV absorption of a sunscreen active in a topical sunscreen composition. The preferred compositions and methods of the present invention use a dibenzoylmethane sunscreen active, a *Keempferia galanga* extract, and a cosmetically acceptable vehicle.

EP 1,269,999 describes a patch having a backing which has been subjected to an ultraviolet-screening processing, the backing usually being of one layer. The ultraviolet-screening processing usually uses an organic ultraviolet absorbent and/or an inorganic ultraviolet-screening agent. The patch can contain in its base a medicine of poor photostability and a non-steroidal anti-inflammatory analgesic.

US 5,000,945 describes a process for stabilising a specific UVB absorbing compound which comprises adding to said UVB absorbing compound at least one specific UVA absorbing compound and also a cosmetic composition and a UV absorber both containing said specific UVB absorbing compound and said specific UVA absorbing compound respectively.

Radschuweit et al: "UV-Induces Formation of Hydrogen Peroxide Based on the Photochemistry of Ketoprofen" Photochemistry and Photobiology, Wylie-Blackwell Publishing Inc, US Volume 73, No 2, 1 February 2001 (pages 119 to 127) describes the fact that ketoprofen as a potent non-steroidal anti-inflammatory drug but that its application to the skin is problematic because of the photosensitising properties of the benzo-phenone moiety. Using capillary electrophoresis with electrochemical detection the authors were able to differentiate the peroxides formed during illumination of KP-containing solutions of linoleic acid the skin damaging effect with regards to cell liability, DNA synthesis efficiency and intra-cellular concentration of peroxides were determined.

EP 1698330 is a patent with filing date of 26.12.2003 and is published on 06.09.2006 (= E-document). This document discloses an adhesive patch comprising a NSAID (ketoprofen in the examples) and BM-DBM.

### Disclosure of Invention

### Problems to be Solved by the Invention

It is an object of the present invention to more reliably prevent photosensitive dermatitis in an external preparation for percutaneous administration containing ketoprofen.

### Means for Solving the Problems

During an intensive investigation by the present inventors in order to attain the above-mentioned object, it was noticed that, with regard to drug-induced photosensitive dermatitis there is phototoxicity, which is caused by a nonimmunological mechanism in which active oxygen generated when a drug is irradiated with sunlight causes tissue/cell damage, and photoallergy, in which a drug haptenated by exposure to sunlight causes a tissue/cell disorder via an immunological mechanism; the former has the possibility of occurring with anybody, irrespective of individual predisposition, if a sufficient dose of UV radiation is received, whereas the latter only affects some individuals who have been sensitized to the allergy by the haptenated drug, the symptoms appearing irrespective of the dose of drug or the intensity of sunlight irradiation, and it is therefore necessary to suppress both of these mechanisms in order to reliably prevent photosensitive dermatitis.

As a result of further investigation based on such a viewpoint, it has been found that, by adding to the preparation a UVA blocker, which, among UV rays, particularly blocks long wavelength UV rays (UVA: wavelength 320 to 400 nm), the photoallergy, which is thought to cause drug-induced photosensitive dermatitis, can be suppressed, and the present invention has thus been accomplished.

That is, the present invention relates an external preparation for percutaneous administration, for use in the prevention of tissue/cell damage caused by photoallergy of a photosensitive non-steroidal anti-inflammatory drug, comprising ketoprofen as photosensitive nonsteroidal anti-inflammatory drug and 4-*tert*-butyl-4'-methoxydibenzoylmethane as UVA blocker, wherein the proportion of 4-*tert*-butyl-4'-methoxydibenzoylmethane as UVA blocker is 0.5 to 20 wt % relative to the entire amount of the composition.

### Effects of the Invention

In the external preparation for percutaneous administration of the present invention, the development of photosensitive dermatitis due to the photosensitive ketoprofen can be outstandingly suppressed. That is, in the present invention, the UVA blocker for blocking UVA by absorption, scattering, etc., suppresses effectively the haptenation of the drug, as well as the occurrence of active oxygen and free radicals from a photosensitive drug, which react with a biological protein to produce adverse effects, both of which are thought to be a cause of the photosensitive dermatitis.

Although external preparations to which a UV absorber is added are known in the art (ref. Patent Publications 1, 3 to 6), in none thereof it is reported that photoallergy is suppressed, and an external preparation for percutaneous administration of ketoprofen having such effects has been realized for the first time by the present invention.

The external preparation for percutaneous administration used in the present invention as described above can exhibit anti-inflammatory and analgesic effects while sufficiently preventing the development of dermatoses such as contact dermatitis or photosensitive dermatitis due to photoallergy, and is expected to be put to practical use as a pharmaceutical having very high safety.

### Best Mode for Carrying Out the Invention

The proportion of UVA blocker in the external preparation for percutaneous administration of the present invention is 0.5 to 20 wt % relative to the entire amount of the composition. If the proportion of UVA blocker is less than the above-mentioned lower limit, the effect in suppressing photoallergy tends not to be exhibited sufficiently, whereas if it exceeds the above-mentioned upper limit, there is a possibility of causing dermatitis or hypersensitivity.

As described above, the present invention more reliably prevents photosensitive dermatitis due to photoallergy in an external preparation for percutaneous administration containing ketoprofen as a medicinal component.

The proportion of ketoprofen in the external preparation for percutaneous administration used in the present invention is 0.1 to 10 wt % relative to the entire amount of the preparation. If the proportion of ketoprofen is less than the above-mentioned lower limit, the anti-inflammatory and analgesic effects tend not be adequately exhibited.

In the present invention, 'to suppress' means that, as a result of the inclusion of the UVA blocker of the present invention, a numerical value related to photoallergy evaluated by a local lymph node test (ref. Example 3), a skin photosensitization test (ref. Example 4), etc. decrease compared with a case in which the UVA blocker is not included. The extent of the decrease (suppression rate) is preferably at least 30%, more preferably at least 40%, yet more preferably at least 50%, and most preferably at least 60%.

It is preferable for the external preparation for percutaneous administration used in the present invention to contain, in addition to the above-mentioned essential components (the above-mentioned NSAID and UVA blocker related to the present invention), a base for each preparation according to the form of the preparation. Examples of the form of the external preparation for percutaneous administration used in the present invention include a patch such as a poultice or a plaster, a gel, a cream, an ointment, and a liniment. The base and a formulation example according to the form of each preparation of the external preparation for percutaneous administration used in the present invention are explained below.

The poultice is first explained. A poultice base used in the poultice described in the present invention is not particularly limited, and is selected from those normally used. Examples of components contained in such a poultice base include a thickener (synthetic water-soluble polymers such as sodium polyacrylate, polyacrylic acid, polyvinyl alcohol, polyvinylpyrrolidone, polyoxyethylene, and polyvinyl methacrylate, natural products such as gum arabic, starch, and gelatin, methyl cellulose, hydroxypropyl cellulose, alginic acid, sodium alginate, ammonium alginate, sodium carboxymethyl cellulose, etc.), a humectant (urea, glycerol, polyethylene glycol, propylene glycol, butylene glycol, sorbitol, etc.), a filler (kaolin, talc, bentonite, epoxy resins, organic acids (citric acid, tartaric acid, maleic acid, maleic anhydride, succinic acid, etc.), calcium, magnesium, aluminum, etc.), water, a solubilization agent (propylene carbonate, crotamiton, diisopropyl adipate, etc.), an irritation inhibitor (diphenhydramine hydrochloride, chlorpheniramine maleate, glycyrrhizinic acid, dexamethasone, betamethasone, fluocinolone acetonide, etc.), and other additives (salicylic acid, methyl salicylate, ethyleneglycol salicylate, 1-menthol, camphor, nonylic acid vanillylamide, red pepper extract, peppermint oil, Azone (registered trademark), etc.), and by adding the above-mentioned essential components to a poultice base formed by mixing components selected from the above, the poultice used in the present invention may be obtained.

A preferred production example (formulation example) of the poultice is now described. That is, 0.1 to 10 parts by weight of the NSAID and 0.01 to 20 parts by weight of the UVA blocker are first mixed with 0.5 to 8 parts by weight of a solubilization agent and dissolved to give a uniform mixture A. Separately, 5 to 20 parts by weight (preferably 10 to 15 parts by weight) of a thickener is mixed with 5 to 40 parts by weight of a humectant and 10 to 80 parts by weight of water and dispersed/dissolved, and 20 parts by weight or less of a filler is further added thereto to give a uniform paste B. Subsequently, mixture A and paste B are mixed to give a uniform paste. The paste so obtained is applied by spreading it on a support by a standard method, and a release cover is then affixed thereon to give the poultice used in the present invention. As the support, a stretchable or non-stretchable support may be used. Specific examples of such a support include those formed from cloth, nonwoven fabric, polyurethane, polyester, polyvinyl acetate, polyvinylidene chloride, polyethylene, polyethylene terephthalate, aluminum sheet, and a composite material thereof. Examples of the release cover include those formed from polyethylene, polypropylene, polyvinyl chloride, polyester, polyvinylidene chloride, and silicone-treated paper.

The plaster is now explained. The plaster base used in the plaster described by the present invention is not particularly limited, and is selected from those normally used. Examples of components contained in such a plaster base include a polymer base (an acrylic composition, that is, a copolymer of a methacrylic acid ester and a vinyl monomer such as acrylonitrile, vinyl acetate, or vinyl propionate, a silicone resin, polyisoprene rubber, natural rubber, acrylic rubber, a styrene-butadiene-styrene block copolymer, a styrene-isoprene-styrene block copolymer, etc.), a fat or a higher fatty acid (almond oil, olive oil, camellia oil, persic oil, peanut oil, olein oil, liquid paraffin, polybutene, etc.), a tackifier (rosin, rosin-modified maleic acid, a hydrogenated rosin ester, etc.), a fatty acid metal salt (zinc undecylenate, zinc stearate, calcium stearate, aluminum stearate, magnesium stearate, sodium stearate, zinc palmitate, zinc myristate, magnesium myristate, sodium laurate, zinc laurate, etc.), an irritation inhibitor, and other additives (salicylic acid, methyl salicylate, ethyleneglycol salicylate, 1-menthol, camphor, nonylic acid vanillylamide, red pepper extract, peppermint oil, Azone (registered trademark), etc.), and by adding the above-mentioned essential components to a plaster base formed by mixing components selected from the above, the plaster used in the present invention may be obtained.

A preferred production example (formulation example) of the plaster is now described. That is, in the case of production being carried out by a hot-melt method, 5 to 40 parts by weight of the polymer base, 20 to 70 parts by weight of the fat or higher fatty acid, 10 to 40 parts by weight of the tackifier, and 0.1 to 10 parts by weight of the fatty acid metal salt are first mixed using a mixing machine such as a kneader or a mixer while heating at 120°C to 160°C, and subsequently 0.1 to 10 parts by weight of ketoprofen and 0.01 to 20 parts by weight of the UVA blocker are added thereto. The mixture so obtained is either spread directly on a support or first spread on a paper, a film, etc. that has been given a release treatment and then compression-transferred so as to cover a desired support. In the case of production being carried out by a solvent method, the components are dissolved in a solvent such as toluene, hexane, or methylene chloride using a mixing machine such as an explosion-proof mixer, the solution so obtained is spread on a paper, film, etc. that has been given a release treatment, the solvent is removed by evaporation using a dryer, and the mixture is then compression-transferred so as to cover a desired support. By affixing a release cover to the coating spread on the support, the plaster used in the present invention is obtained. Specific examples of such a support include those formed from cloth, nonwoven fabric, polyurethane, polyester, polyvinyl acetate, polyvinylidene chloride, polyethylene, polyethylene terephthalate, aluminum sheet, and a composite material thereof. Examples of the release cover include polyethylene, polypropylene, polyvinyl chloride, polyester, polyvinylidene chloride, and silicone-treated paper.

The ointment is now explained. An ointment base used in the ointment described by the present invention is not particularly limited, and is selected from those normally used. Examples of components contained in such an ointment base include a higher fatty acid or an ester thereof (adipic acid, myristic acid, palmitic acid, stearic acid, oleic acid, an adipic acid ester, an myristic acid ester, a palmitic acid ester, diethyl sebacate, hexyl laurate, cetyl isooctanoate, etc.), a wax (spermaceti, beeswax, ceresine, etc.), a surfactant (a polyoxyethylene alkyl ether phosphoric acid ester, etc.), a fatty alcohol (cetanol, stearyl alcohol, cetostearyl alcohol, etc.), a silicone oil (dimethyl polysiloxane, methyl phenyl polysiloxane, glycol methyl polysiloxane, silicone glycol polymer, etc.), a hydrocarbon (hydrophilic petrolatum, white petrolatum, purified lanolin, liquid paraffin, etc.), water, an absorption enhancer (propylene carbonate, diisopropyl adipate, crotamiton, Azone (registered trademark), etc.), a moisturizing agent (glycerol, propylene glycol, butylene glycol, sorbitol, etc.), an irritation inhibitor, and other additives (salicylic acid, methyl salicylate, ethyleneglycol salicylate, 1-menthol, camphor, peppermint oil, etc.), and by adding the above-mentioned essential components to an ointment base formed by mixing components selected from the above, the ointment used in the present invention may be obtained.

A preferred production example (formulation example) of the ointment is now described. That is, 0.1 to 10 parts by weight of the NSAID and 0.01 to 20 parts by weight of the UVA blocker are first mixed with 5 to 15 parts by weight of a higher fatty acid ester and 1 to 10 parts by weight of a surfactant at room temperature or while heating, 4 to 10 parts by weight of a wax and 50 to 90 parts by weight of a hydrocarbon are added thereto, and the mixture is heated and maintained at 50°C to 100°C. After all of the components become a transparent solution, they are mixed uniformly using a homomixer. Following this, the mixture so obtained is cooled to room temperature while stirring to give the ointment used in the present invention.

The gel is now explained. A gel base used in the gel described by the present invention is not particularly limited, and is selected from those normally used. Examples of components contained in such a gel base include a lower alcohol (ethanol, isopropanol, etc.), water, a gelling agent (carboxyvinyl polymer, hydroxyethyl cellulose, hydroxypropyl cellulose, methyl cellulose, ethyl cellulose, carboxymethyl cellulose, propylene glycol alginate, etc.), a neutralizing agent (triethanolamine, diisopropanolamine, sodium hydroxide, etc.), a surfactant (sorbitan sesquioleate, sorbitan trioleate, sorbitan monooleate, sorbitan monostearate, sorbitan monolaurate, polyethylene glycol monostearate, polyoxyethylene nonylphenyl ether, polyoxyethylene cetyl ether, polyoxyethylene lauryl ether, etc.), an absorption enhancer (propylene carbonate, diethyl sebacate, diisopropyl adipate, crotamiton, Azone (registered trademark), propylene glycol, etc.), a solubilizer (a lower alcohol such as ethanol or isopropanol, a fatty alcohol such as cetyl alcohol, stearyl alcohol, batyl alcohol, behenyl alcohol, oleyl alcohol, hexadecyl alcohol, or octyldodecanol, a fatty acid ester such as isopropyl myristate, octyldodecyl myristate, cetyl myristate, myristyl myristate, diethyl sebacate, diisopropyl sebacate, diisopropyl adipate, oleyl oleate, hexyl laurate, cetyl isooctanoate, a medium chain fatty acid triglyceride, or a propylene glycol fatty acid ester, N-methyl-2-pyrrolidone, triacetin, benzyl alcohol, lanolin alcohol, 1-menthyl glyceryl ether, etc.), a glycol (glycerol, propylene glycol, polyethylene glycol, polypropylene glycol, sorbitol, 1,3-butylene glycol, dipropylene glycol, etc.), an irritation inhibitor, and other additives (salicylic acid, methyl salicylate, ethyleneglycol salicylate, 1-menthol, camphor, peppermint oil, etc.), and by adding the above-mentioned essential components to a gel base formed by mixing components selected from the above, the gel used in the present invention may be obtained.

A preferred production method (formulation example) for the gel is now described. That is, 0.5 to 5 parts by weight of a gelling agent is first added to 55 parts by weight or less of water and swollen to give a swollen product A. Separately, 0.1 to 10 parts by weight of ketoprofen and 0.01 to 20 parts by weight of the UVA blocker are dissolved or suspended in 0.1 to 10 parts by weight of a solubilizer, and this is further dissolved in a mixture of 40 parts by weight or less of a glycol and 60 parts by weight or less of a lower alcohol to give a dissolved product B. Subsequently, after the dissolved product B is added to the swollen product A, a neutralizing agent is added thereto so as to adjust the pH value to 4 to 7, thus giving the gel used in the present invention.

The cream is now explained. A cream base used in the cream described by the present invention is not particularly limited, and is selected from those normally used. Examples of components contained in such a cream base include a higher fatty acid ester (an adipic acid ester, a myristic acid ester, a palmitic acid ester, diethyl sebacate, hexyl laurate, cetyl isooctanoate, etc.), a lower alcohol (ethanol, isopropanol, etc.), a carbohydrate (liquid paraffin, squalane, etc.), a polyhydric alcohol (propylene glycol, 1,3-butylene glycol, etc.), a fatty alcohol (2-hexyldecanol, cetanol, 2-octyldodecanol, etc.), an emulsifier (a polyoxyethylene alkyl ether, a fatty acid ester, a polyethylene glycol fatty acid ester, etc.), a preservative (a paraoxybenzoic acid ester, etc.), an absorption enhancer (propylene carbonate, diethyl sebacate, diisopropyl adipate, crotamiton, Azone (registered trademark), propylene glycol, etc.), an irritation inhibitor, and other additives (salicylic acid, methyl salicylate, ethyleneglycol salicylate, 1-menthol, camphor, peppermint oil, etc.), and by adding the above-mentioned essential components to a cream base formed by mixing components selected from the above, the cream used in the present invention may be obtained. Furthermore, in order to obtain a gel cream, which has properties between a cream and a gel, a gelling agent (carboxyvinyl polymer, hydroxyethyl cellulose, hydroxypropyl cellulose, methyl cellulose, ethyl cellulose, carboxymethyl cellulose, propylene glycol alginate, etc.) and a neutralizing agent (triethanolamine, diisopropanolamine, sodium hydroxide, etc.) may be added to the above-mentioned cream so as to adjust the pH to 4 to 8 (preferably 5 to 6.5), thus giving the gel cream used in the present invention.

A preferred production example (formulation example) of the gel cream is now described. That is, 0.1 to 10 parts by weight of ketoprofen and 0.01 to 20 parts by weight of the UVA blocker are dissolved in a mixture of 25 parts by weight or less of a higher fatty acid ester and 40 parts by weight or less of a lower alcohol and, furthermore, 0.5 parts by weight or less of a preservative and 5 parts by weight or less of an emulsifier are added thereto to give a mixture A. Separately, a gelling agent is added to water so as to give a concentration of 0.5 to 5 parts by weight and swollen to give a swollen product B. Subsequently, the swollen product B is added to the mixture A, the mixture is emulsified uniformly by means of a homomixer, and a neutralizing agent is then added to the emulsified product so obtained so as to adjust the pH value to 4 to 8, thus giving the gel cream used in the present invention.

The liniment is now explained. A liniment base used in the liniment described by the present invention is not particularly limited, and is selected from those normally used. Examples of components contained in such a liniment base include a mixture of 10 to 70 parts by weight of an alcohol (a monohydric alcohol such as ethanol, propanol, or isopropanol, a polyhydric alcohol such as polyethylene glycol, propylene glycol, or butylene glycol, etc.), 60 parts by weight or less of a fatty acid ester (various esters of adipic acid, sebacic acid, or myristic acid, etc.), and 10 parts by weight or less of a surfactant (a polyoxyethylene alkyl ether, polyoxyethylene hardened castor oil, etc.), and by adding 0.1 to 10 parts by weight of ketoprofen and 0.01 to 20 parts by weight of the UVA blocker to such a liniment base, the liniment used in the present invention may be obtained. In addition, in the liniment used in the present invention, a neutralizing agent for adjusting the pH, a thicknening agent such as methyl cellulose, an irritation inhibitor, another additive (salicylic acid, methyl salicylate, ethyleneglycol salicylate, 1-menthol, camphor, peppermint oil, red pepper extract, nonylic acid vanillylamide, crotamiton, Azone (registered trademark), propylene carbonate, diisopropyl adipate, etc.), etc. may be added as necessary.

The above-mentioned formulation examples and production examples are simply examples, and the liniment used in the present invention can of course be obtained by a known production method for a liniment. Furthermore, with regard to the composition, by replacing a medicinal component of a known liniment with ketoprofen, and adding the UVA blocker, the liniment used in the present invention may easily be obtained.

Preferred embodiments of the bases and the formulation examples according to each form of the external preparation for percutaneous administration used in the present invention are explained above, but the preparation forms and the formulation examples are not limited to the above, and the order of mixing each component is not particularly limited either. For example, in the formulation of conventionally known eye drops or the formulation of a conventionally known aerosol, by replacing a medicinal component with 0.1 to 10 parts by weight of ketoprofen, and further adding 0.01 to 20 parts by weight of the UVA blocker, eye drops or an aerosol used in the present invention may be obtained.

Furthermore, in the external preparation for percutaneous administration used in the present invention, an antioxidant may be added to the above-mentioned formulation. As such an antioxidant, a phenol derivative such as *tert-*butylhydroxyanisole, di-*tert*-butylhydroxytoluene, thymol, or propyl gallate, tocopherol and ester derivatives thereof, ascorbic acid and ester derivatives thereof, etc. are preferable. Such an antioxidant may be used singly or in a combination of two or more types. The proportion thereof is not particularly limited, but it is preferably 0 to 10 wt % relative to the entire amount of the preparation, and more preferably 0 to 5 wt %.

### [Examples]

The present invention is more specifically explained below by reference to Examples, which should not be construed as being limited to the Examples below. Furthermore, in the Examples below, '%' means 'wt %' unless otherwise noted.

### Example 1 (In vitro phototoxicity test) REFERENCE EXAMPLE

The experiment below was carried out in accordance with "Guidelines for basic biological tests of medical materials and device", part VII "Hemolysis Test". That is, each of test substances {4-*tert*-butyl-4'-methoxydibenzoylmethane (BM-DBM), terephthalylidene-3,3'-dicamphor-10,10'-disulfonic acid (TP-DCS), *n*-hexyl 2-(4-diethylamino-2-hydroxybenzoyl)benzoate (DHB-BH)} and ketoprofen (KP) were dissolved in *N,N*-dimethylformamide (DMF), and then diluted with phosphate buffered saline (PBS) to give a concentration of twice the test concentration. 1 mL fractions thereof were added to a 24-well multiwell plate, and to each was added 1 mL of PBS, to which had been added 40 µL of heparin-containing rabbit venous blood (whole blood) per 5 mL, to give a reaction solution. The final concentration of KP was 50 µg/mL. As a control, a well into which DMF alone was added instead of the test substance solution was prepared. A cover was removed from the plate into which the reaction solutions had been put, and it was irradiated at 37°C with UVA at 7.5 J/cm² using UV irradiation equipment (UVP Lamp Fixture, UVP). When irradiating with UV rays, a glass plate 3 mm inthickness was placed between a light source and the reaction solution, thus cutting out UV rays with a wavelength of 320 nm or less. The incubation time, including the irradiation time, was 60 minutes. After the reaction was completed it was centrifuged, 100 µL of the supernatant so obtained was placed in a 96-well assay plate, and absorbance at 540 nm (Abs₅₄₀) was measured and used as a hemolysis index. The influence of each test substance on the photohemolysis reaction due to KP was evaluated using the Abs₅₄₀, and a 50% inhibition dose (ID₅₀ value) was calculated from a hemolysis inhibition rate (%) relative to the control group. The results obtained are given in Table 1.

**[Table 1]**

| Test Substance | ID₅₀ Value (µg/mL) |
|---|---|
| BM-DBM | 2.7 |
| TP-DCS | 5.4 |
| DHB-BH | 2.1 |

As is clear from the results shown in Table 1, it has been confirmed that, in the external preparation for percutaneous administration containing ketoprofen as a medicinal component, adding a UVA blocker enables phototoxicity to be suppressed outstandingly.

### Example 2 (In vivo phototoxicity test) REFERENCE EXAMPLE

The experiment below was carried out by partially refining the method of Gerberick et al., (Food Chem. Toxicol., 27, 813-819 (1989)). That is, a Balb/c mouse (female, 9 to 11 weeks old) was used as a test animal, each of the test substances {4-*tert*-butyl-4'-methoxydibenzoylmethane (BM-DBM), 2-ethylhexyl dimethoxybenzylidenedioxoimidazolidine propionate (DB-DIH), terephthalylidene-3,3'-dicamphor-10,10'-disulfonic acid (TP-DCS), *n*-hexyl 2-(4-diethylamino-2-hydroxybenzoyl)benzoate (DHB-BH)} and 2% ketoprofen (KP) were dissolved in ethanol and applied to the auricle, and it was irradiated with UVA at 40 J/cm². 24 hours after UVA irradiation, the ear thickness was measured, and an increase from the ear thickness prior to the start of the test was calculated. The effect in suppressing phototoxicity due to KP was evaluated for each test substance using as an index the degree of suppression of the increase in ear thickness due to KP, that is, the ear swelling suppression rate (%) toward the KP group. The results obtained are given in Table 2 and Table 3.

**[Table 2]**

| Test Substance (Concentration: 0.5%) | Ear Swelling Suppression Rate (%) |
|---|---|
| BM-DBM | 98 |
| DB-DIH | 34 |
| TP-DCS | 65 |
| DHB-BH | 84 |

**[Table 3]**

| Test Substance (Concentration: %) | Ear Swelling Suppression Rate (%) |
|---|---|
| BM-DBM (0.1) | 41 |
| BM-DBM (0.3) | 81 |
| BM-DBM (0.5) | 98 |

As is clear from Table 2 and Table 3, it has been confirmed that in the external preparation for percutaneous administration containing ketoprofen as a medicinal component, adding the UVA blocker enables phototoxicity to be suppressed outstandingly.

### Example 3 (In vivo photoallergy test 1)

A KP photoallergy detection test was carried out by refining the Murine Local Lymph Node Assay (LLNA) proposed by the Interagency Coordinating Committee on the Validation of Alternative Methods (ICCVAM). That is, a Balb/c mouse (female, 8 to 12 weeks old) was used as a test animal, a solution of a test substance {4-*tert*-butyl-4'-methoxydibenzoylmethane (BM-DBM)} and 2% ketoprofen (KP) prepared in acetone - olive oil (4:1, v/v) was applied to the back of the pinna, and it was irradiated with UVA at 20 J/cm². This sensitization induction treatment was carried out for 3 days in succession. 5 days after starting the sensitization, 20 µCi per mouse of ³H-methyl thymidine (³H-TdR)/PBS solution was injected into the tail vein, and 5 hours later an auricular lymph node was removed. The removed lymph node was made into a single lymph cell suspension using a cell strainer, and after washing twice with PBS was then allowed to stand in a 5% trichloroacetic acid (TCA) solution at 4°C overnight to thus make DNA precipitate. After the precipitate was suspended in 1 mL of 5% TCA solution, the radioactivity was measured using a liquid scintillation counter. The effect of the test substance in suppressing photoallergy due to KP was evaluated using as an index the degree of suppression of the increase in ³H-TdR uptake by KP, that is, the ³H-TdR uptake suppression rate (%) toward the KP group. The results obtained are given in Table 4.

**[Table 4]**

| BM-DBM Concentration (%) | ³H-TDR Uptake Suppression Rate (%) |
|---|---|
| 0.06 | 30 |
| 0.13 | 51 |
| 0.25 | 24 |
| 0.50 | 94 |
| 1.00 | 99 |

As is clear from the results in Table 4, it has been confirmed that, in the external preparation for percutaneous administration containing ketoprofen as a medicinal component, adding the UVA blocker enables photoallergy to be suppressed outstandingly.

### Example 4 (In vivo photoallergy test 2)

A skin photoallergy test using a guinea pig was carried out by partially refining the Adjuvant and Strip Method of Sato et al., (The Nishinihon Journal of Dermatology, 42, 831-837 (1980)). That is, the back of the neck of a Hartley white female guinea pig (a group of 8 animals) was depilated, an adjuvant was administered to four corners of 2 × 2 cm, a 2% ethanol solution of ketoprofen (KP) or a solution of 2% KP + 2% test substance {4-tert-butyl-4'-methoxydibenzoylmethane (BM-DBM)} was openly applied to the 2 × 2 cm area, and 1 hour later it was irradiated with UVA (10 J/cm²). This sensitization induction treatment was carried out for 5 days in succession. 3 weeks after starting the sensitization, the back of the hip was depilated, the same solution as for the sensitization was openly applied to a 2 × 2 cm area, and 1 hour later it was irradiated with UVA (10 J/cm²) to thus carry out a photoinduction. A skin reaction 24 and 48 hours after the irradiation was evaluated in accordance with the above-mentioned criteria of Sato et al. The effect of BM-DBM in suppressing photoallergy due to KP was evaluated by erythema and swelling suppression rates (%) toward the KP group. The results obtained are given in Table 5.

**[Table 5]**

| Test Substance | Erythema/Swelling Suppression Rate (%) | |
|---|---|---|
| | After 24 Hr | After 48 Hr |
| BM-DBM | 69 | 74 |

As is clear from the results shown in Table 5, it has been confirmed that, in the external preparation for percutaneous administration containing ketoprofen as a medicinal component, adding the UVA blocker enables photoallergy to be suppressed outstandingly.

Formulation Examples of the external preparation for percutaneous administration of the present invention are now described, but the present invention should not be construed as being limited to the formulations below.

### Formulation Example 1 (Plaster)

16 parts by weight of a styrene-isoprene-styrene block copolymer (SIS5200P: manufactured by JSR), 10 parts by weight of polyisobutylene (L-100: manufactured by Exxon Mobil), 19 parts by weight of a petroleum resin (Arkon P-70: manufactured by Arakawa Chemical Industries, Ltd.), 45 parts by weight of a liquid paraffin (Crystol J-352: manufactured by Esso Petroleum Ltd.), and 1.99 parts by weight of a synthetic aluminum silicate were heated under an atmosphere of nitrogen gas while stirring to give a melt product (Step A). The temperature when stirring was 110°C to 200°C, and the stirring time was 30 to 120 minutes. Subsequently, 3 parts by weight of crotamiton, 0.01 parts by weight of 4-*tert*-butyl-4'-methoxydibenzoylmethane, 2 parts by weight of ketoprofen, and 3 parts by weight of 1-menthol were added to the above melt product in a temperature range of 110°C to 200°C while stirring, and the mixture was mixed for 5 to 30 minutes to give a plaster base as a uniform melt product (Step B). After the base was spread at a weight of 1 g/70 cm² on a silicone-treated polyester film, it was covered with a polyester woven fabric, compression-transferred, and cut into a desired size, thus giving a plaster of the present invention.

### Formulation Examples 2 to 10 (Plaster) REFERENCE EXAMPLE

Preparation was carried out in the same manner as in Formulation Example 1 using the components and the proportions shown in Table 6, and plasters were thus obtained.

**[Table 6]**

| | Form. Ex.2 | Form. Ex.3 | Form. Ex.4 | Form. Ex.5 | Form. Ex.6 | Form. Ex.7 | Form. Ex.8 | Form. Ex.9 | Form. Ex.10 |
|---|---|---|---|---|---|---|---|---|---|
| Ketoprofen* | 2.00 | 2.00 | 2.00 | 2.00 | 2.00 | 2.00 | 2.00 | 2.00 | |
| Diclofenac* | | | | | | | | | 2.00 |
| 4-*tert*-Butyl-4'-methoxydibenzoylmethane* | | 5.00 | 20.00 | 3.00 | 3.00 | | 3.00 | | 5.00 |
| *n*-Hexyl 2-(4-diethylamino-2-hydroxybenzoyl)benzoate* | 3.00 | | | | | 3.00 | | | |
| Zinc Oxide* | | | | | | | | 5.00 | |
| Styrene-isoprene-styrene block copolymer** | 15.00 | 17.00 | 15.00 | 15.00 | 15.00 | 15.00 | 15.00 | 15.00 | 26.00 |
| Polyisobutylene** | 9.00 | 9.00 | 8.00 | 10.00 | 10.00 | 8.00 | 8.00 | 7.00 | 9.00 |
| Petroleum resin** | | | | | | | | | 20.00 |
| Liquid paraffin** | 50.00 | 50.00 | 40.00 | 50.00 | 45.00 | 50.00 | 50.00 | 50.00 | 35.00 |
| Synthetic aluminum silicate** | | | | | | 1.49 | | | 3.00 |
| Maleic Acid hydrogenated rosin ester** | 14.00 | 11.00 | 10.00 | 13.00 | | 15.00 | | 10.00 | |
| Sodium stearate** | 2.00 | | | | | | 4.00 | | |
| Zinc stearate** | | 1.00 | | | 2.00 | | | | |
| Magnesium stearate** | | | 1.00 | | | | | 2.00 | |
| Calcium stearate** | | | | 1.99 | | | | | |
| Hydrogenated rosin glycerol ester** | | | | | 13.00 | | 10.00 | | |
| Crotamiton* | | | | 2.00 | 2.00 | | | 1.00 | |
| 1-Menthol* | 3.00 | 3.00 | 3.00 | 3.00 | 3.00 | 3.00 | 3.00 | 3.00 | |
| Diethyl sebacate* | 2.00 | | | | | | | | |
| Propylene glycol* | | 2.00 | | | | | | | |
| Diisopropyl adipate* | | | 1.00 | | | 1.00 | | | |
| Propyl gallate* | | | | 0.01 | | 0.01 | 2.00 | 2.00 | |
| Di-*tert*-butylhydroxytoluene* | | | | | 5.00 | 1.50 | 3.00 | 3.00 | |
| Dipropylene glycol* | | | | | | | 1.50 | | 1.00 |
| Total amount (wt%) | 100.00 | 100.00 | 100.00 | 100.00 | 100.00 | 100.00 | 100.00 | 100.00 | 100.00 |

| | | | | | | | | | |
|---|---|---|---|---|---|---|---|---|---|
| * Added in step B ** Mixed in step A | | | | | | | | | |

### Formulation Example 11 (Plaster)

50 parts by weight of 2-ethylhexyl acrylate, 25 parts by weight of methoxyethyl acrylate, 14.7 parts by weight of vinyl acetate, 0.3 parts by weight of azobisisobutyronitrile, 3 parts by weight of synthetic aluminum silicate, and 100 parts by weight of ethyl acetate were placed in a reaction vessel, a polymerization was started by heating to 65°C under an atmosphere of nitrogen, the reaction was carried out for 10 hours, and it was further aged at 80°C for 2 hours to give a copolymer solution. 5 parts by weight of 4-tert-butyl-4'-methoxydibenzoylmethane and 2 parts by weight of ketoprofen were added to the copolymer solution so obtained to give a plaster base as a uniform mixed solution. The base thus obtained was spread at a weight of 1 g/70 cm² on a silicone-treated polyester film; after the ethyl acetate was evaporated by hot air it was covered with a polyester woven fabric, compression-transferred, and cut into a desired size, thus giving a plaster used in the present invention.

### Formulation Example 12 (Plaster)

45 parts by weight of 2-ethylhexyl acrylate, 25 parts by weight of methoxyethyl acrylate, 12 parts by weight of vinylpyrrolidone, 1 part by weight of benzoyl peroxide, 3 parts by weight of synthetic aluminum silicate, 5 parts by weight of 4-*tert*-butyl-4'-methoxydibenzoylmethane, 5 parts by weight of di-*tert*-butylhydroxytoluene, and 100 parts by weight of ethyl acetate were placed in a reaction vessel, a polymerization was started by heating to 65°C under an atmosphere of nitrogen, the reaction was carried out for 10 hours, and it was further aged at 80°C for 2 hours to give a copolymer solution. 4 parts by weight of ketoprofen was added to the copolymer solution so obtained to give a plaster base as a uniform mixed solution. The base thus obtained was spread at a weight of 1 g/70 cm² on a silicone-treated polyester film; after the ethyl acetate was evaporated by hot air it was covered with a polyester woven fabric, compression-transferred, and cut into a desired size, thus giving a plaster used in the present invention.

### Formulation Example 13 (Poultice)

0.3 parts by weight of ketoprofen and 1 part by weight of 4-*tert*-butyl-4'-methoxydibenzoylmethane were mixed and dissolved in 0.5 parts by weight of crotamiton to thus give a uniform mixture A. Separately, 6 parts by weight of sodium polyacrylate, 3 parts by weight of polyacrylic acid, 2 parts by weight of gelatin, and 1 part by weight of polyvinyl alcohol were mixed and dispersed/dissolved in 20 parts by weight of glycerol, 5 parts by weight of sorbitol, and 59.6 parts by weight of water and, furthermore, 0.5 parts by weight of synthetic aluminum cinnamate, 0.1 parts by weight of dihydroxyaluminum aminoacetate, 0.3 parts by weight of magnesium metasilicate aluminate, and 0.2 parts by weight of tartaric acid were added thereto to give a uniform paste B. Subsequently, the mixture A was added to and mixed with the paste B to give a uniform paste. After the paste so obtained was spread on a polyester nonwoven fabric, a polypropylene film was affixed thereon to give a poultice used in the present invention.

### Formulation Examples 14 to 16 (Poultice) REFERENCE EXAMPLE

Preparation was carried out in the same manner as in Formulation Example 13 using the components and the proportions shown in Table 7, and poultices were obtained.

**[Table 7]**

| (wt%) | | | |
|---|---|---|---|
| | Form. Ex. 14 | Form. Ex.15 | Form. Ex. 16 |
| Diclofenac | 0.5 | 5.0 | |
| Ibuprofen | | | 10.0 |
| 4-*tert*-Butyl-4'-methoxydibenzoylmethane | 3.0 | | |
| 2-(2H-Benzotriazol-2-yl)-4-methyl-6-[2-methyl-3-[1,3,3,3-tetramethyl-1-[(trimethylsilyl)oxy]disiloxanyl]propyl]phenol | | 10.0 | 15.0 |
| Crotamiton | 3.0 | 5.0 | 8.0 |
| 1-Menthol | 0.5 | 0.5 | 0.5 |
| Sodium polyacrylate | 6.0 | 6.0 | 6.0 |
| Polyacrylic acid | 3.0 | 3.0 | 3.0 |
| Gelatin | 2.0 | 2.0 | 2.0 |
| Polyvinyl alcohol | 1.0 | 1.0 | 1.0 |
| Glycerol | 20.0 | 20.0 | 20.0 |
| Sorbitol | 5.0 | 5.0 | 5.0 |
| Synthetic aluminum silicate | 0.5 | 0.5 | 0.5 |
| Dihydroxyaluminum aminoacetate | 0.1 | 0.1 | 0.1 |
| Magnesium metasilicate aluminate | 0.3 | 0.3 | 0.3 |
| Tartaric Acid | 0.2 | 0.2 | 0.2 |
| Water | 54.9 | 41.4 | 28.4 |

### Formulation Example 17 (ointment)

2 parts by weight of ketoprofen and 0.4 parts by weight of 4-*tert*-butyl-4'-methoxydibenzoylmethane were mixed with 4.7 parts by weight of diethyl sebacate and 4.4 parts by weight of glycerol monooleate at room temperature or with heating, 6.8 parts by weight of beeswax, 8.1 parts by weight of liquid paraffin, and 73.6 parts by weight of white petrolatum were added thereto, and the mixture was heated and maintained at 50°C to 100°C. After all the components had become a transparent solution, they were mixed uniformly using a homomixer. Subsequently, by cooling the mixture so obtained to room temperature while stirring, an ointment used in the present invention was obtained.

### Formulation Examples 18 to 22 (Ointment) REFERENCE EXAMPLE

Preparation was carried out in the same manner as in Formulation Example 17 using the components and the proportions shown in Table 8, and ointments were obtained.

**[Table 8]**

| | Form. Ex. 18 | Form. Ex. 19 | Form. Ex.20 | Form. Ex.21 | Form. Ex.22 |
|---|---|---|---|---|---|
| Ketoprofen | 2.0 | | | | |
| Suprofen | | 3.0 | 3.0 | | |
| Piroxicam | | | | 1.0 | 1.0 |
| 4-*tert*-Butyl-4'-methoxy benzoylmethane | 2.1 | | | | |
| Terephthalylidene-3,3'-dicamphor-10,10'-disulfonic acid | | 0.2 | 5.3 | | |
| *n*-Hexyl 2-(4-diethylamino-2-hydroxybenzoyl)benzoate | | | | 1.4 | 17.6 |
| Diethyl sebacate | 4.7 | 4.7 | 4.7 | 4.7 | 4.7 |
| Glycerol monooleate | 4.4 | 4.4 | 4.4 | 4.4 | 4.4 |
| Beeswax | 6.8 | 6.8 | 6.8 | 6.8 | 6.8 |
| Liquid Paraffin | 8.1 | 8.1 | 8.1 | 8.1 | 8.1 |
| White petrolatum | 71.9 | 72.8 | 67.7 | 73.6 | 57.4 |
| Total amount (wt%) | 100.0 | 100.0 | 100.0 | 100.0 | 100.0 |

### Formulation Example 23 (Gel) REFERENCE EXAMPLE

1.8 parts by weight of a carboxyvinyl polymer was added to 30.2 parts by weight of purified water and swollen to give a swollen product A. Separately, 3 parts by weight of tiaprofenic acid, 2.5 parts by weight of isostearyl 4-hydroxy-3-methoxycinnamate, and 0.5 parts by weight of dibutylhydroxytoluene were dissolved in a mixture of 13.7 parts by weight of propylene glycol and 42.5 parts by weight of absolute ethanol to thus give a dissolved product B. Subsequently, the dissolved product B was added to the swollen product A, and 0.9 parts by weight of triethanolamine was then added thereto so as to adjust the pH, thus giving a gel.

### Formulation Examples 24 to 30 (Gel) REFERENCE EXAMPLE

Preparation was carried out in the same manner as in Formulation Example 23 using the components and the proportions shown in Table 9, and gels were obtained.

**[Table 9]**

| | Form. Ex. 24 | Form. Ex. 25 | Form. Ex. 26 | Form. Ex. 27 | Form. Ex. 28 | Form. Ex. 29 | Form. Ex. 30 |
|---|---|---|---|---|---|---|---|
| Tiaprofenic acid | 3.00 | | | | | | |
| Tolmetin | | 1.00 | 1.00 | | | | |
| Naproxen | | | | 5.00 | 5.00 | | |
| Diflunisal | | | | | | 2.00 | 2.00 |
| Isostearyl 4-hydroxy-3-methoxycinnamate | 8.40 | | | | | | |
| 4-Hydroxy-3-methoxycinnamic acid | | 0.04 | 3.90 | | | | |
| 2-Ethylhexyl dimethoxybenzylidenedioxoimidazo lidinepropionate | | | | 4.20 | 12.20 | | |
| 1-(3,4-Dimethoxyphenyl) 4,4-dimethyl-1,3-pentanedione | | | | | | 1.60 | 7.30 |
| Absolute ethanol | 42.50 | 42.50 | 42.50 | 42.50 | 42.50 | 42.50 | 42.50 |
| Propylene glycol | 13.70 | 13.70 | 13.70 | 13.70 | 13.70 | 13.70 | 13.70 |
| Diisopropyl adipate | 4.90 | 4.90 | 4.90 | 4.90 | 4.90 | 4.90 | 4.90 |
| Dibutylhydroxytoluene | 0.50 | 0.50 | 0.50 | 0.50 | 0.50 | 0.50 | 0.50 |
| Carboxyvinyl polymer | 1.80 | 1.80 | 1.80 | 1.80 | 1.80 | 1.80 | 1.80 |
| Triethanolamine | 0.90 | 0.90 | 0.90 | 0.90 | 0.90 | 0.90 | 0.90 |
| Purified Water | 24.30 | 34.66 | 30.80 | 26.50 | 18.50 | 32.10 | 26.40 |
| Total amount (wt%) | 100.00 | 100.00 | 100.00 | 100.00 | 100.00 | 100.00 | 100.00 |

### Formulation Examples 31 to 38 (Cream) REFERENCE EXAMPLE

By mixing the components shown in Table 10 at the proportions shown in the same table, creams were obtained.

**[Table 10]**

| | Form. Ex. 31 | Form. Ex. 32 | Form. Ex. 33 | Form. Ex. 34 | Form. Ex. 35 | Form. Ex. 36 | Form. Ex. 37 | Form. Ex. 38 |
|---|---|---|---|---|---|---|---|---|
| Carprofen | 3.00 | 3.00 | | | | | | |
| Benoxaprofen | | | 1.00 | 1.00 | | | | |
| Benzydamine | | | | | 5.00 | 5.00 | | |
| Azapropazone | | | | | | | 2.00 | 2.00 |
| Terephthalylidene-3,3'-dicamphor-10,10'-disulfonic acid | 2.50 | 8.40 | | | | | | |
| *n*-Hexyl 2-(4-diethylamino-2-hydroxybenzoyl)benzoate | | | 0.04 | 3.90 | | | | |
| Isostearyl 4-hydroxy-3-methoxy cinnamate | | | | | 4.20 | 12.20 | | |
| Zinc oxide | | | | | | | 1.60 | 7.30 |
| Diethyl sebacate | 3.50 | 3.50 | 3.50 | 3.50 | 3.50 | 3.50 | 3.50 | 3.50 |
| Cetanol | 9.60 | 9.60 | 9.60 | 9.60 | 9.60 | 9.60 | 9.60 | 9.60 |
| Liquid paraffin | 6.80 | 6.80 | 6.80 | 6.80 | 6.80 | 6.80 | 6.80 | 6.80 |
| White petrolatum | 12.40 | 12.40 | 12.40 | 12.40 | 12.40 | 12.40 | 12.40 | 12.40 |
| Dibutylhydroxytoluene | 0.30 | 0.30 | 0.30 | 0.30 | 0.30 | 0.30 | 0.30 | 0.30 |
| Polyoxyethylene stearyl ether | 4.30 | 4.30 | 4.30 | 4.30 | 4.30 | 4.30 | 4.30 | 4.30 |
| Methyl paraoxybenzoate | 0.20 | 0.20 | 0.20 | 0.20 | 0.20 | 0.20 | 0.20 | 0.20 |
| Purified water | 57.40 | 51.50 | 61.86 | 58.00 | 53.70 | 45.70 | 59.30 | 53.60 |
| Total amount (wt%) | 100.00 | 100.00 | 100.00 | 100.00 | 100.00 | 100.00 | 100.00 | 100.00 |

### Formulation Examples 39 to 46 (Liniment) REFERENCE EXAMPLE

By mixing the components shown in Table 11 at the proportions shown in the same table, liniments were obtained.

**[Table 11]**

| | Form. Ex. 39 | Form. Ex. 40 | Form. Ex. 41 | Form. Ex. 42 | Form. Ex. 43 | Form. Ex. 44 | Form. Ex. 45 | Form. Ex. 46 |
|---|---|---|---|---|---|---|---|---|
| Diclofenac | 5.0 | 5.0 | | | | | | |
| Naproxen | | | 2.0 | 2.0 | | | | |
| Ketoprofen | | | | | 3.0 | 3.0 | | |
| Diflunisal | | | | | | | 1.0 | 1.0 |
| *n*-Hexyl 2-(4-diethylamino-2-hydroxybenzoyl)benzoate | 0.7 | 3.2 | | | | | | |
| 4-*tert-*Butyl-4'-methoxybenzoylmethane | | | 4.8 | 13.9 | | | | |
| 2-Ethylhexyl dimethoxybenzylidenedioxoim idazolidinepropionate | | | | | 2.7 | 9.4 | | |
| Isostearyl 4-hydroxy-3-methoxycinnamate | | | | | | | 1.7 | 7.3 |
| Absolute ethanol | 48.8 | 48.8 | 48.8 | 48.8 | 48.8 | 48.8 | 48.8 | 48.8 |
| Propylene glycol | 7.8 | 7.8 | 7.8 | 7.8 | 7.8 | 7.8 | 7.8 | 7.8 |
| Diethyl sebacate | 3.1 | 3.1 | 3.1 | 3.1 | 3.1 | 3.1 | 3.1 | 3.1 |
| Polyoxyethylene hardened castor oil | 2.2 | 2.2 | 2.2 | 2.2 | 2.2 | 2.2 | 2.2 | 2.2 |
| Dibutylhydroxytoluene | 0.2 | 0.2 | 0.2 | 0.2 | 0.2 | 0.2 | 0.2 | 0.2 |
| Purified water | 32.2 | 29.7 | 31.1 | 22.0 | 32.2 | 25.5 | 35.2 | 29.6 |
| Total amount (wt%) | 100.0 | 100.0 | 100.0 | 100.0 | 100.0 | 100.0 | 100.0 | 100.0 |

### Industrial Applicability

As hereinbefore described, in accordance with the present invention, in the external preparation for percutaneous administration containing ketoprofen as a medicinal component that might cause photosensitive dermatitis, it becomes possible to exhibit anti-inflammatory and analgesic effects while more reliably preventing photosensitive dermatitis due to photoallergy, and application as a pharmaceutical having very high safety is expected.

## Claims

1. An external preparation for percutaneous administration, for use in the prevention of tissue/cell damage caused by photoallergy of a photosensitive non-steroidal anti-inflammatory drug, comprising ketoprofen as photosensitive nonsteroidal anti-inflammatory drug and 4-*tert-*butyl-4'-methoxydibenzoylmethane as UVA blocker, wherein the proportion of 4-*tert*-butyl-4'-methoxydibenzoylmethane as UVA blocker is 0.5 to 20 wt % relative to the entire amount of the composition.

2. The external preparation according to Claim 1 for use according to claim 1,
wherein the proportion of ketoprofen is 0.1 to 10 wt % relative to the entire amount of the composition.

3. The external preparation according to Claim 1 or 2 for use according to claims 1 or 2, wherein the suppression rate evaluated by *in vivo* skin photoallergy test upon irradiation of UVA compared with a case in which UVA blocker is not included is at least 60 %.

4. The external preparation according to any one of claims 1 to 3 for use according to claims 1-3, wherein the form of the preparation is selected from the group consisting of a patch, a poultice, a plaster, a gel, a cream, an ointment, and a liniment.

5. The external preparation according to any one of claims 1 to 3 for use according to claims 1-3, wherein the form of the preparation is selected from the group consisting of a patch, a poultice, a plaster, an ointment, and a liniment.

## Patentansprüche

1. Eine äußerliches Präparat zur perkutanen Verabreichung zur Verwendung bei der Prävention von Gewebe-/Zellschäden, die durch Lichtallergie eines lichtempfindlichen nichtsteroidalen entzündungshemmenden Mittels verursacht werden, beinhaltend Ketoprofen als lichtempfindliches nichtsteroidales entzündungshemmendes Mittel und 4-*tert*-Butyl-4'-methoxydibenzoylmethan als UVA-Blocker, wobei der Anteil von 4-*tert-*Butyl-4'-methoxydibenzoylmethan als UVA-Blocker 0,5 bis 20 Gew.-% in Bezug auf die Gesamtmenge der Zusammensetzung beträgt.

2. Äußerliches Präparat gemäß Anspruch 1 zur Verwendung gemäß Anspruch 1, wobei der Anteil von Ketoprofen 0,1 bis 10 Gew.-% in Bezug auf die Gesamtmenge der Zusammensetzung beträgt.

3. Äußerliches Präparat gemäß Anspruch 1 oder 2 zur Verwendung gemäß den Ansprüchen 1 oder 2, wobei die Unterdrückungsrate bei Bestrahlung mit UVA, evaluiert durch einen *In-vivo*-Hautlichtallergietest, im Vergleich zu einem Fall, in dem kein UVA-Blocker eingeschlossen ist, mindestens 60 % beträgt.

4. Äußerliches Präparat gemäß einem der Ansprüche 1 bis 3 zur Verwendung gemäß den Ansprüchen 1-3, wobei die Form des Präparates aus der Gruppe ausgewählt ist, die aus einem Pflaster, einer Packung, einem Heftpflaster, einem Gel, einer Creme, einer Salbe und einem Liniment besteht.

5. Äußerliches Präparat gemäß einem der Ansprüche 1 bis 3 zur Verwendung gemäß den Ansprüchen 1-3, wobei die Form des Präparates aus der Gruppe ausgewählt ist, die aus einem Pflaster, einer Packung, einem Heftpflaster, einer Salbe und einem Liniment besteht.

## Revendications

1. Une préparation externe pour administration percutanée, destinée à être utilisée dans la prévention de dommages tissulaires/cellulaires causés par une photoallergie d'un médicament anti-inflammatoire non stéroïdien photosensible, comprenant du kétoprofène en tant que médicament anti-inflammatoire non stéroïdien photosensible et du 4-*tert*-butyl-4'-méthoxydibenzoylméthane en tant qu'écran bloquant les UVA, dans laquelle la proportion de 4-*tert*-butyl-4'-méthoxydibenzoylméthane en tant qu'écran bloquant les UVA est de 0,5 à 20 % en poids relativement à la quantité totale de la composition.

2. La préparation externe selon la revendication 1 destinée à être utilisée selon la revendication 1, dans laquelle la proportion de kétoprofène est de 0,1 à 10 % en poids relativement à la quantité totale de la composition.

3. La préparation externe selon la revendication 1 ou la revendication 2 destinée à être utilisée selon les revendications 1 ou 2, dans laquelle le taux de suppression évalué par test de photoallergie cutané *in vivo* après irradiation d'UVA comparé à un cas dans lequel l'écran bloquant les UVA n'est pas inclus est d'au moins 60 %.

4. La préparation externe selon l'une quelconque des revendications 1 à 3 destinée à être utilisée selon les revendications 1 à 3, dans laquelle la forme de la préparation est sélectionnée dans le groupe consistant en un timbre, un cataplasme, un emplâtre, un gel, une crème, un onguent, et un liniment.

5. La préparation externe selon l'une quelconque des revendications 1 à 3 destinée à être utilisée selon les revendications 1 à 3, dans laquelle la forme de la préparation est sélectionnée dans le groupe consistant en un timbre, un cataplasme, un emplâtre, un onguent, et un liniment.
